(19) Europäisches Patentamt
European Patent Office
Office européen des brevets

(11) Publication number: **0 058 840**
**B1**

(12) **EUROPEAN PATENT SPECIFICATION**

(45) Date of publication of patent specification: **03.07.85**

(51) Int. Cl.$^4$: **G 03 G 5/06,** C 07 D 209/80

(21) Application number: **82100687.1**

(22) Date of filing: **01.02.82**

(54) **Electron donor compounds and photoconductive charge transport materials.**

(30) Priority: **23.02.81 US 237068**
**23.02.81 US 236892**

(43) Date of publication of application:
**01.09.82 Bulletin 82/35**

(45) Publication of the grant of the patent:
**03.07.85 Bulletin 85/27**

(84) Designated Contracting States:
**DE FR GB IT**

(56) References cited:
**DE-A-2 063 227**
**FR-A-2 303 316**
**GB-A-2 019 868**
**US-A-3 206 306**
**US-A-3 832 172**
**US-A-4 092 161**

**XEROX DISCLOSURE JOURNAL, vol. 3, no. 1, January-February 1978, page 7; M. STOLKA: "Electrophotographic photoreceptor"**

(73) Proprietor: **MINNESOTA MINING AND MANUFACTURING COMPANY**
**3M Center, P.O. Box 33427**
**St. Paul, MN 55133 (US)**

(72) Inventor: **Stofko, John J., Jr.**
**2501 Hudson Road P.O. Box 33427**
**St. Paul Minnesota 55133 (US)**
Inventor: **Kneipp, Kenneth G.**
**2501 Hudson Road P.O. Box 33427**
**St. Paul Minnesota 55133 (US)**
Inventor: **Sonnonstine, Terry J.**
**2501 Hudson Road P.O. Box 33427**
**St. Paul Minnesota 55133 (US)**
Inventor: **Leichter, Louis M.**
**2501 Hudson Road P.O. Box 33427**
**St. Paul Minnesota 55133 (US)**

(74) Representative: **Baillie, Iain Cameron et al**
**c/o Ladas & Parry Isartorplatz 5**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

Technical Field

The present invention relates to novel compounds which are electron donor compounds and novel photo-conductive charge transporting layers comprising these novel compounds in binders. These layers are particularly useful in imaging systems such as electrophotography or electroradiography.

Background of the Art

The technology of electrophotography is commercially well established. A wide variety of processes and apparatus are used, although they have many characteristics in common. One of the more common forms of this technology involves the use of a plate having a photoconductive insulating layer, generally coated on a conductive layer. Imaging is effected by first uniformly electrostatically charging the surface of the photoconductive layer and then exposing the charged layer to an image or pattern of activating electromagnetic radiation, usually visible or ultraviolet radiation. This exposure selectively enables the charge in the irradiated areas of the photoconductive insulator to dissipate. The charge which remains in the nonirradiated areas forms a latent image which may be further processed to form a more permanent record of the exposing image or pattern. The most common form of additional processing involves the attraction of particles of material selectively to the charged areas and fusing them to the photoconductive layer or transferring the particles in their imagewise distribution to another surface to which they are more permanently bound by an adhesive or by fusion of the particles themselves. A common electrophotographic construction comprises, in sequence, a substrate, a conductive layer, and a photoconductive insulating layer.

GB—A— 2 019 868 discloses Carbazolylmethane compounds of the formula

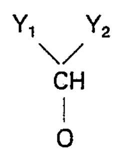

(1)

wherein one of $Y_1$, $Y_2$ and Q represents a 3-carbazolyl radical of the formula

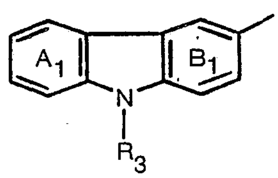

(1a)

and each of the two independently represents an amino-substituted phenyl radical or a 3-carbazolyl radical of Formula 1a and Q also represents, *inter alia*, alkyl of 1 to 10 carbon atoms, aryl or aralkyl radicals. Ring $A_1$ can be substituted by at least one fused benzene nucleus. The compounds can be used as colour formers in pressure-sensitive or heat-sensitive recording material.

Fr—A— 2 303 316 discloses as colouring agents compounds having two or more quinoid groups joined through a cyclopropane or a cyclobutane bridging structure.

Typical classes of photoconductive materials useful in electrophotography include 1) inorganic crystalline photoconductors such as cadmium sulfide, cadmium sulfoselenide, cadmium selenide, zinc sulfide, zinc oxide, and mixtures thereof, 2) inorganic photoconductive glasses such as amorphous selenium, selenium alloys, and selenium-arsenic, and 3) organic photoconductors such as phthalocyanine pigments and polyvinyl carbazole, with or without binders and additives which extend their range of spectral sensitivity. These systems are well known in the art. For example, U.S. Patent No. 3,877,935 discusses various problems associated with the crystalline and amorphous classes of photoconductors and shows the use of polynuclear quinone pigments in a binder as a photoconductive layer. U.S. Patent No. 3,824,099 shows the use of squaric acid methine sensitizing dyes and triaryl pyrazoline charge transport materials as an electrophotographic construction. Cadmium sulfoselenide plates are shown in U.S Patent No. 3,764,315, and one of the original disclosures of the use of poly-N-vinylcarbazole as a photoconductive insulating layer is provided in U.S. Patent No. 3,037,861. A number of diverse organic photoconductors have been disclosed since the development of the carbazole class of photoconductors such as quinones and anthrones (e.g., Hayashi et al., *Bull. Chem. Soc. Japan,* vol. 39, (1966) pp. 1670—1673), but the carbazoles have continued to attract the greatest attention.

Problems particularly associated with the use of carbazoles as a positive charge transporting material which is capable of supporting the injection of photoexcited holes from a photoconductive layer and is capable of transporting the injected holes also exist in this area of technology. The carbazole condensates with aldehydes as shown in U.S. Patent No. 4,025,341 have a tendency to oligomerize. This oligomerization

can cause a number of problems. The oligomers formed are not of a uniform molecular weight and carbazole content. This creates problems in purification and can create undesirable variations in photoconductive or charge transport properties. Triaryl methanes including a carbazole moiety (as shown in Xerox Disclosure Journal, Vol. 3, No. 1, Jan/Feb 1978, page 7) also tend to be sensitive to oxidation which converts them to an ionic species which will not act as a photoconductive insulator but rather will act as a conductor.

Japanese Patent Publication 52—34735 discloses carbazole organic photoconductor materials which may have substituents thereon which would inherently prevent oligomerization of the carbazoles. This is not recongnized in the disclosure and the carbazoles would still be subject to oxidation problems.

## Summary of the Invention

A novel class of electronically active organic donor compounds has the formula:

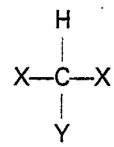

where X is

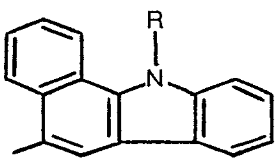

wherein R is an aliphatic, aromatic, or mixed aliphatic-aromatic group and Y is an aliphatic, aromatic, heterocyclic, or mixed aliphatic-aromatic group. For example, R and Y may be independently selected from alkyl groups, benzyl groups, phenyl groups, naphthyl groups, anthracyl groups, etc., with such various substituents as alkoxy groups, amine groups, alkyl groups, hydroxyl groups, and halogen atoms thereon.

These compounds have been found to be electron donor compounds and are useful in forming photoconductive electrically active insulating layers. They may be combined with polymeric binder materials to form photoconductive insulating layers and the compounds have a reduced sensitivity to oxygen and oligomerization.

## Detailed Description of the Invention

The novel compounds of the present invention are bis (benzocarbazoles) which may be represented by the formula

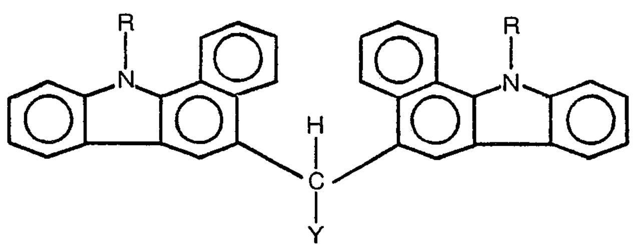

wherein R and Y are independently selected from the group aliphatic, aromatic, heterocyclic, and mixed aliphatic-aromatic groups, and the benzocarbazole rings can be unsubstituted or substituted.

All of the compounds of the present invention may be synthesized by reacting the appropriate N-substituted benzo[a]carbazole

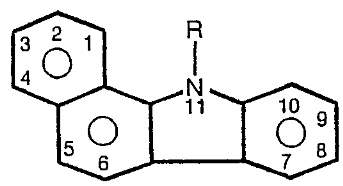

with the correspondingly appropriate aldehyde:

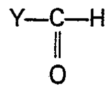

This process can be carried out in a solvent (e.g., ethanol) in the presence of an acid (e.g., HCl) catalyst. The reaction product may be isolated by simple filtration and washing. For example, in the reaction of 11-ethylbenzo[a]carbazole with benzaldehyde in ethanol in the presence of HCl as a catalyst, because of the preferential reaction of the aldehyde at the 5-position of the 11-benzo[a]carbazole and the insolubility of the reaction product:

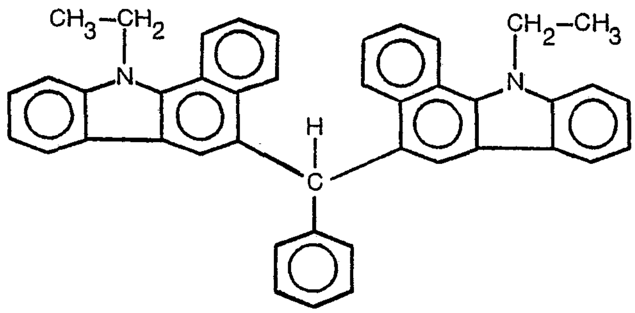

in ethanol, no oligomeric species are formed such as occur in a similar reaction with N-ethyl-carbazole. The reaction product is also stabilized against oxidation of the methine group by the rings ortho to the point at which the methine group is bonded to the benzocarbazole nucleus.

The benzocarbazole groups may bear substituents in positions other than N-substituents. Substituted benzocarbazole groups which are used as reagents in the synthesis of the bis(benzocarbazole)methanes of the present invention are well reported in the literature. The only significant limitation on the substitution of the reagents is that they not be substituted in the coupling position 5. The substituents known in the art include alkyl, alkoxy, nitro, halogen, phenyl, naphthyl, benzyl, etc. The phenyl rings may be fused to the benzocarbazole by sharing two carbon atoms e.g., 5,6), three or four carbon atoms (e.g., 6,7 and bridging atoms). Heterocyclic rings and additional substituents on the substituted groups (e.g., -bromobutyl, -ethoxyethyl (i.e., a diethyl ether substituent), may be present. Preferably the substituents have fewer than 20 carbon atoms. The main effect of substitution is in the solubility characteristics of the compounds of the present invention. Substitution is most readily effected on position 8 of the benzocarbazole, but as noted above, substitution of the other positions is well known in the art. Substituted and non-substituted benzocarbazoles shall be distinguished, according to the practice of the present invention, by referring to formulae of compounds that may contain substitution as 'groups' and by referring to compounds which may not contain substitution as moieties.

R may, as previously stated, by selected from aliphatic, aromatic and mixed aliphatic-aromatic groups. These groups may or may not be substituted. If they are substituted, it would be preferred that they be electron donating substituents although electron withdrawing substituents may be tolerated. Preferably R is selected from alkyl groups of 1 to 20 carbon atoms, preferably n-alkyl groups of 2 to 20 carbon atoms, aryl groups such as phenyl or naphthyl groups, with phenyl groups preferred, alkaryl groups, for example benzyl groups, and allyl groups. Where the term 'group' is used anywhere in the practice of the present invention, as opposed to the term 'radical', the possibility of substitution is specifically intended to be included within the definition of that term. For example, n-alkyl radical may be only of the formula $—(CH_2)_n—CH_3$ while n-alkyl group may have hydrogen atoms on the n-alkyl radical substituted with other moieties such as halogen atoms, hydroxyl radicals, alkoxy radicals, alkyl radicals, amine radicals, cyano radicals, etc. Specific examples of useful R moieties are ethyl, n-butyl, n-propyl, 4-methoxybutyl, 3-chloropropyl, 8-hydroxyoctyl, phenyl, benzyl, allyl, p-ethylphenyl, m-tert-butylnaphthayl, p-diethylaminophenyl, stearyl, dodecyl, etc. R preferably has fewer than 20 carbon atoms, but may have up to 30 or more carbon atoms. The main influence of this group, except where electronic induction occurs because of a change of the nature of this group, is in the solubility of the compound.

Y may, as previously stated, be selected from aliphatic, aromatic, and mixed aliphatic-aromatic groups. These groups may or may not be substituted. Examples of useful moieties are methyl, ethyl, n-pentyl, nonyl, stearyl, tolyl, anisyl (m-, p-, and o-), p-chlorobenzyl, o-bromobenzyl, p-hydroxybenzyl, veratryl, isobutyl, terphthalyl, p-octyloxybenzyl, p-dimethylaminophenyl, t-butyl, etc. Preferred Y moieties are phenyl, tolyl, anisyl, and benzyl groups because of their availability. As with group R, the main influence of this group, except with regard to electron induction effects, is on the solubility of the compounds. Preferably Y has 20 or fewer carbon atoms, but up to 30 may be readily tolerated.

Various binder materials known in the art are useful with the electronically active donor compounds of the present invention. It is of course preferred that the binder be essentially optically transparent or at least

transparent to the wavelengths of radiation to which the compounds (sensitized or not) are sensitive. Amongst the useful binders are poly(vinyl chloride), poly(siloxanes), poly(vinyl butyral), poly(vinyl acetate), styrene/acrylonitrile copolymers, polyacrylates, polymethacrylates, polycarbonates, polyepoxides, polyurethanes, polyamides, polyethers, polyesters, polyolefins as well as block, graft, random, and alternating polymers, copolymers, terpolymers and mixtures thereof and the like. The binders are preferably electrically inactive themselves. The preferred polymeric binders are polycarbonates, polyesters, and styrene/acrylonitrile copolymers. Coating aids, lubricants, surface active agents, and others adjuvants may be added to the composition.

For use of the materials of the present invention as electrophotographic layers, the organic electron donor compounds should be present as at least 20 percent by weight of the composition. Preferably the donor compound should be present as at least 25 or 35 percent by weight of the layer, and may comprise up to 100% by weight of the layer, excluding of course the sensitizer dye. The sensitizing dyes should be used in amounts which will increase the sensitivity of the composition. This is defined as an effective sensitizing amount of dye. Ordinaryly amounts of up to 10% by weight dye may be used, but certain constructions can be envisaged with as much as 90% by weight of dye and 10% by weight of organic electron donor compounds. Amounts of dye as small as 0.005 percent by weight can be useful. More preferred concentration ranges are between 0.05 and 5 percent by weight.

The photosensitive materials of the present invention may also be useful as photoconductive toners, photovoltaic devices, organic semiconductors, and the like, and may use concentrations or organic electronic donor compounds as low as 5 percent by weight.

Spectral sensitizers for photoconductive systems, as known in the art such as for example U.S. Patent No. 3,037,861 are useful herein. These materials are generically classified as mineral acids, organic carboxylic acids, organic sulfonic acids, organic phosphonic acids, nitrophenols, acid anhydrides, metal halides, boron halides, gumones, aldehydes, and ketones. The benzocarbazoles of the present invention can also be sensitized with 1) disulfone dyes, 2) quinoxaline dyes, 3) polyquinoid and polyanthraquinoid dyes, and 4) indolenine dyes respectively.

It has been surprisingly noted that the benzocarbazole-aldehyde condensation products of the present invention are better charge transport materials than the corresponding benzocarbazoles by themselves. This is surprising because it is the benzocarbazole nucleus which is the electronically active portion of both molecules. Even when benzocarbazoles were used in reasonably higher molecular proportions to the binder than were the condensates, the condensates would still perform better.

These and other aspects of the present invention will be shown in the following examples.

### Example 1

Synthesis of bis-5,5′-(n-ethylbenzo[a]carbazolyl)phenylmethane.

Into a round bottom flask equipped with a reflux condenser and a mechanical stirrer were added 22.4 grams (0.1 mole) of N-ethylbenzo[a]carbazole and 5.3 grams (0.05 mole) of benzaldehyde. Two hundred milliliters of ethanol acidified with 8 ml of concentrated hydrochloric acid were then added. The mixture was stirred at reflux under a nitrogen atmosphere for sixteen hours. The insoluble, pure white product was isolated by filtration, washed with 100 ml of ethanol, and dried in a vacuum oven. The yield was 95% of the theoretic calculation.

### Examples 2—17

In a manner substantially identical to that of the previous example, electronically active electron donor compounds of the present invention were obtained by condensing N-ethylbenzo[a]carbazole with each of the following aldehydes in equimolar replacement for the benzaldehyde:

2. p-tolualdehyde
3. m-tolualdehyde
4. o-tolualdehyde
5. p-anisaldehyde
6. m-anisaldehyde
7. o-anisaldehyde
8. p-chlorobenzaldehyde
9. p-bromobenzaldehyde
10. o-bromobenzaldehyde
11. p-hydroxybenzaldehyde
12. α-naphthaldehyde
13. veratraldhyde
14. p-octyloxybenzaldehyde
15. *iso*-butyraldehyde
16. n-nonylaldehyde
17. terphthaldehyde

Examples 18—20

In a manner substantially identical to that of Example 1, the following combinations of carbazoles and aldehydes were used to synthesize compounds of the present invention.

18. benzo[a]carbazole and benzaldehyde
19. N-ethyldibenzo[a,g]carbazole and benzaldehyde
20. N-ethyl-8-methoxybenzo[a]carbazole and benzaldehyde

The addition of any of the compounds produced in Examples 1—20 to electrically inert polymeric binders formed positive charge transport layers. These layers could be coated on photoconductive charge generation layers and were capable of supporting injected photogenerated holes from the photoconductive layer and allowed the transport of these holes through the transport layer to selectively discharge the surface charge.

Examples 21—25

An electrophotographic plate was constructed of three layers. A photoconductive charge generation layer was coated onto a conductive glass substrate by conventional vapor deposition of a 0.5 micrometer thick amorphous selenium/tellurium alloy (95 atomic percent selenium and 5 atomic percent tellurium) using a resistive heater. The charge generation layer was then overcoated with solutions of electron donor compounds in organic polymer. In each of these examples the amounts of the electron donor compound and resin were varied to keep the molar ratio of the two constant with respect to the molar ratio of the 40/60 weight ratio of N-ethylbenzocarbazole to poly (4,4'-isopropylidenediphenylene carbonate) used in Example 1. This solution was provided as a 10% solids solution in 1,2-dichloroethane and coated out at 75 microns wet thickness to provide a dry charge transport layer 7 µm thick. The samples were air dried at room temperature for at least twelve hours before evaluation.

Each of the plates were negatively charged by a screened corona charging device and then exposed to a 90 foot candle tungsten illumination. The surface potential was monitored using an electrostatic voltmeter. The photodischarges at that level of exposure were essentially instantaneous to a residual potential voltage level. The performance of various devices, with the electron donor compounds noted, are shown in the following Table. The percent discharge was determined as the ratio of the initial potential ($V_1$) minus the residual potential ($V_r$) divided by the initial potential and multiplied by 100%. That is,

$$\frac{V_I - V_r}{V_I} \times 100\%.$$

TABLE

| Example | Compound | Initial Potential (volts) | Residual Potential (volts) | Discharge (%) |
|---|---|---|---|---|
| 21 | 6 | 608 | 74 | 88 |
| 22 | 5 | 670 | 110 | 84 |
| 23 | 1 | 618 | 118 | 81 |
| 24 | 12 | 593 | 126 | 79 |
| 25 | N-ethylbenzo [a]carbazole | 569 | 232 | 59 |

As an alternate means of evaluation, the field dependence of the initial discharge rates under low level illumination conditions at 500 nm were measured. Since the initial discharge rate is proportional to the photoinjection efficiency under emission limited conditions, a direct measure of the field dependence of the hole photoinjection efficiency can be obtained.

An electrophotographic device produced according to this example exhibited improved xerographic performance and was capable of use in forming visible images by fixation of toner particles.

An electrophotographic device was prepared by first vacuum depositing about 1.0 µm thick amorphous Se/Te (94 atomic percent Se/6 atomic percent Te, 25 ppm chlorine) onto a glow discharge cleaned, flexible, aluminized polyester web (100 µm thick) using resistive heating techniques. The Se/Te charge generation layer was then overcoated with a ten percent solids solution [50%] (wt) 1,2-dichloroethane/50% (wt) methylene dichloride] of [40% (wt) electron donor compound of examples 1—20/60% (wt) Vitel PE200 copolyester of terephthalic acid, isophthalic acid and ethylene glycol. About a 75 wet coating thickness produced a dry transport layer coating thickness of 5—7 µm.

An electrophotographic device produced according to this example exhibited improved xerographic performance and was preferred for use in forming visible images.

Examples 26—33

The effect of varying the substituent R on the donor compounds of the present invention were evaluated. The following materials were formed by condensation of the described materials.

| Compound | Carbazole | Aldehyde |
|---|---|---|
| A | N-allylbenzo[a]carbazole | benzaldehyde |
| B | N-benzylbenzo[a]carbazole | benzaldehyde |
| C | N-phenylbenzo[a]carbazole | benzaldehyde |
| D | N-propylbenzo[a]carbazole | benzaldehyde |
| E | N-ethylbenzo[a]carbazole | p-isopropylbenzaldehyde |

Electrophotographic devices were formed on a polyethyleneterephthalate substrate bearing an aluminum layer thereon. A one micrometer layer of Perylene Red (3,5-dimethylphenyl Perylene Red) was vapor deposited onto the aluminium layer and insulating photoconductive charge transport layers according to the present invention was coated on at about 5 mils ($1.25 \times 10^{-4}$m) wet thickness and dried. The layers comprised sixty percent by weight of a polymer (VITEL® PE200 Polyester) and forty percent by weight of the charge transport donor compound:

26. Bis-benzocarbazolephenylmethane (Compound 1)
27. p-methoxy-bis-benzocarbazolephenylmethane (Compound 21)
28. Compound E
29. 20% Bis-benzocarbazolephenylmethane (Compound 1) and 20% Compound D
30. Compound D
31. Compound A
32. Compound C
33. Compound B

The sensitometric data from these elements are shown in the following Table. T represents the thickness of the coating in micrometers, Decay is the percent of dark decay, Speed is the amount of light in foot-candle-seconds [1 foot = 0,3 m] necessary to reach $V_{o/2}$ and $T_r$ is the time constant for the voltage drop during exposure as expressed in the relationship

$$V = V_r e^{-t/T_r}$$

wherein V is the voltage at any time, $V_r$ is the residual voltage, e is the natural logarithm base, and t is the length of time of the exposure.

| Ex. | $V_1$ | $V_o$ | T | Decay | Speed | $V_r$ | $T_r$ | Remarks |
|---|---|---|---|---|---|---|---|---|
| 26 | 338 | 325 | 5.7 | 3.8 | 0.828 | 33.6 | 4.95 | 1,4 |
| 27 | 386 | 368 | 6.4 | 4.7 | 0.565 | 25.9 | 1.89 | 1,5 |
| 28 | 604 | 572 | 8.6 | 5.3 | 5.11 | 117 | 5.28 | 3,5 |
| 29 | 652 | 636 | 14.2 | 2.5 | 1.24 | 110 | 6.86 | 3,7 |
| 30 | 572 | 556 | 8.5 | 2.8 | 1.49 | 117 | 15.9 | 2,6 |
| 31 | 428 | 412 | 7.0 | 3.7 | 0.911 | 49.5 | 7.20 | 1,7 |
| 32 | 920 | 902 | 8.5 | 2.0 | — | 755 | 7.50 | 2,7,8 |
| 33 | 620 | 600 | 9.3 | 3.2 | 1.87 | 197 | 23.0 | 2,7 |

Remarks
1. Coating solution 10% solids in 50/50 dichloroethane/methylene dichloride.
2. Coating solvent of 1, but with 16% solids.
3. Coating solvent of 1 and 2, but with 20% solids.
4. Coating solvent 50/50 dichloroethane/methylene dichloride.
5. Coating solvent 50/50 methylethylketone/toluene.
6. Coating solvent 2/1 methylethylketone/toluene.
7. Coating solvent dichloroethane.
8. Large residual potential obscured white light response.

Example 34

In a further embodiment of an electrophotographic device, the charge generation layer was prepared by vacuum deposition of about 1.0 µm thick arsenic triselenide ($As_2Se_3$) onto a glow discharge cleaned flexible, aluminized polyester web (100 µm thick) using resistive heating techniques. The $As_2Se_3$ charge generation layer was then overcoated with a ten percent solids solution in [50% (wt) 1,2-dichloroethane/50% (wt) methylene dichloride] of [40% (wt) of the electron donor compound of Example 1/60% (wt) polyester]. About a 5 mil (125 µm) wet coating thickness produced a dry transport layer with a coating thickness of 8—10 µm.

The electrophotographic device produced according to this example exhibited improved xerographic performance and can be utilized in forming visible images.

The electron donor compounds of the present invention can be sensitized by spectral sensitizing dyes and in particular by polyquinoid sensitizers which are a part of the present invention are disclosed in U.S. Patents Nos. 4,134,764 and 4,205,005. These references teach polyquinoid dyes (including anthraquinoid dyes) in photoconductive binders as sensitizer dyes.

The dyes may be represented by the formulae:

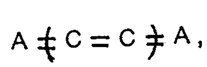

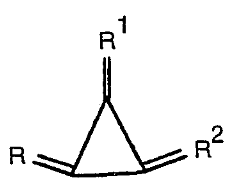

and

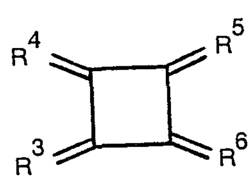

wherein

A is selected from quinoid and anthraquinoid groups,

R and $R^1$ are selected from quinoid and anthraquinoid groups,

$R^2$ is selected from quinoid group, anthraquinoid group, and an oxygen atom,

$R^3$ is a quinoid group, and

$R^4$, $R^5$ and $R^6$ are selected from the class consisting of quinoid groups and oxygen atoms with the proviso that at least one of $R^4$, $R^5$ and $R^6$ must be a quinoid group.

The term quinoid group refers to a quinoid ring, i.e.:

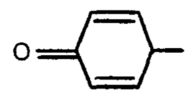

These quinoid groups may bear substituents which should be no more electron withdrawing than chlorine in positions ortho to the quinoid carbonyl group. That is, referred to the following formula, substituents $X_1$ and $X_2$ may be independently positioned only as shown below:

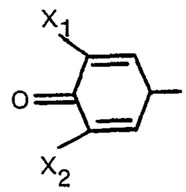

wherein $X_1$ and $X_2$ are groups no more electron withdrawing than chlorine.

One or two substituents may be present in place of hydrogen atoms. Such substituents may include, for example, alkyl and alkoxy groups (straight or branched, preferably having from 1 to 20 carbon atoms), phenyl, phenoxy, halophenyl, alkyl and alkoxy substituted phenyl (having 1 to 10 carbon atoms on the substituent groups), amino, iodo, bromo, chloro, carboxyl, carbanyl and amido groups.

For use of the materials of the present invention as electrophotographic layers, the organic electron donor compounds should be present as at least 20 percent by weight of the composition. Preferably the donor compound should be present as at least 25 or 35 percent by weight of the layer, and may comprise up to 100 percent by weight of the layer, excluding, of course, the sensitizer dye. The sensitizing dyes should be used in amounts which will increase the sensitivity of the composition. This is defined as an effective sensitizing amount of dye. Ordinarily amounts of up to 10 percent by weight dye may be used, but certain constructions can be envisaged with as much as 90 percent by weight of dye and 10 percent by weight of organic electron donor compounds. Amounts of dye as small as 0.005 percent by weight can be useful. More preferred concentration ranges are between 0.05 and 5 percent by weight.

Example 35

A bulk sensitized photoreceptor was prepared by coating a solution consisting of 15 percent by weight solids (5.2% diquinoanthraquinocyclopropane, 47.4% by weight of the compound prepared in Example 1, and 47.4% of a polycarbonate resin) were coated at about $1 \times 10^{-4}$m wet thickness onto an aluminum vapor coated polyethyleneterephthalate film. The sample was air dried at 85°C for 15 minutes. The photoreceptor charged to 720 volts under positive corona charging and the charge was reduced to about 360 volts with little dark decay by an exposure of 0.54 foot-candle-seconds to a tungsten lamp. The device was also found to display high charge acceptance, low dark decay, and negligible fatique upon cycling.

Example 36

A bilayer photoreceptor was prepared by overcoating a solution of triquinocyclopropane and polyvinylbutyral (50/50) as a 15 percent by weight solution in 1,2-dichloroethane onto an aluminized polyester film coated with a fifty/fifty percent solid solution of bis(N-ethyl[a]benzocarbazolyl)phenyl methane and polycarbonate resin. The sample was air dried overnight at room temperature. The device displayed high charge acceptance, low dark decay, negligible fatique on cycling, and the ability to discharge completely.

## Claims

1. An electrically active donor compound of the formula

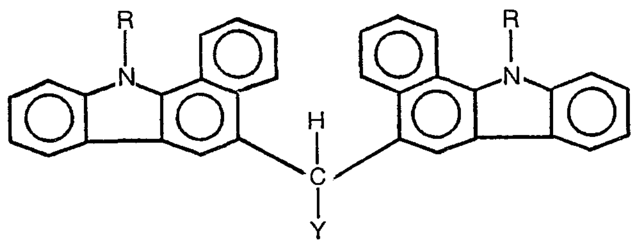

wherein R and Y are independently selected from the group aliphatic, aromatic, hetercyclic, and mixed aliphatic-aromatic groups, and the benzocarbazole rings can be unsubstituted or substituted.

2. A compound according to claim 1 wherein the benzocarbazole rings are unsubstituted and R and Y are independently selected from aliphatic, aromatic and mixed aliphatic-aromatic groups.

3. A compound according to claim 1 wherein Y in an aromatic group.

4. A compound according to claim 1 wherein R in an alkyl group of from 1 to 20 carbon atoms.

5. A compound according to claim 1 wherein Y in an aromatic group and R is an alkyl group of 2 to 20 carbon atoms, a phenyl group, a-naphthyl group, or a benzyl group.

6. A compound according to claim 1 wherein Y is phenyl and R is n-alkyl of 2 to 20 carbon atoms.

7. A compound according to any of claims 1 to 6 sensitized by an effective amount of a polyquinoid dye selected from the class consisting of:

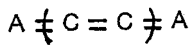

,

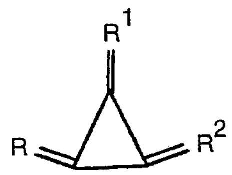

and

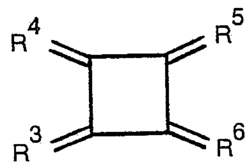

wherein

A is selected from quinoid and anthraquinoid groups,

R and $R^1$ are selected from quinoid and anthraquinoid groups,

$R^2$ is selected from quinoid group, anthraquinoid group, and an oxygen atom,

$R^3$ is a quinoid group, and

$R^4$, $R^5$ and $R^6$ are selected from the class consisting of quinoid groups and oxygen atoms with the proviso that at least one of $R^4$, $R^5$ and $R^6$ must be a quinoid group.

8. A electronically active photoconductive insulating layer comprising a polymeric binder and a compound according to claims 1, 2, 3, 4 5 or 6 or a sensitized compound according to claim 7.

9. A photoconductive element comprising, in sequence, a conductive layer, a charge generating layer, and a photoconductive insulating layer according to claim 8.

10. A photoconductive element comprising a conductive substrate and a layer according to claim 8 wherein R is selected from the class consisting of an alkyl group of 2 to 20 carbon atoms, phenyl group, naphthyl group, or benzyl group.

11. An imaging process comprising providing an electrical charge on an element comprising in sequence a conductive layer, a photoconductive charge generating layer and a photoconductive insulating layer according to claim 8, irradiating said charged element with a pattern of radiation to selectively cause areas of said charge to be conducted to said conductive layer, and selectively attracting material to either the charged or uncharged areas of said element to form an image.

12. The process of claim 11 wherein said image is transferred to another surface by contact therewith.

**Revendications**

1. Un composé donneur électriquement actif de formule

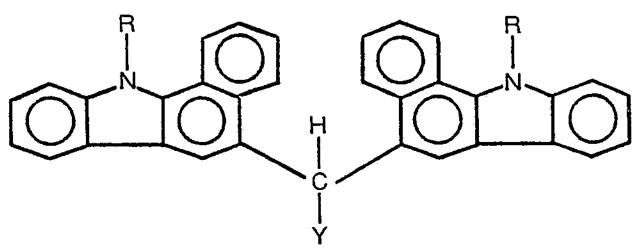

dans laquelle R et Y sont independamment choisis dans le groupe constitué par les groupes aliphatiques, aromatiques, hétérocycliques et aliphatiques-aromatiques mixtes, et les cycles benzocarbazoles peuvent être non substitués ou substitués.

2. Un composé selon la revendication 1, dans lequel les cycles benzocarbazoles sont non substitués, et R et Y sont indépendamment choisis parmi les groupes aliphatiques, aromatiques et aliphatiques-aromatiques mixtes.

3. Un composé selon la revendication 1, dans lequel Y est un groupe aromatique.

4. Un composé selon la revendication 1, dans lequel R est un groupe alkyle de 1 à 20 atomes de carbone.

5. Un composé selon la revendication 1, dans lequel Y est un groupe aromatique et R est un groupe alkyle de 2 à 20 atomes de carbone, un groupe phényle, un groupe naphtyle ou un groupe benzyle.

6. Un composé selon la revendication 1, dans lequel Y est un phényle et R est un n-alkyle de 2 à 20 atomes de carbone.

7. Un composé selon l'une quelconque des revendications 1 à 6, sensibilisé par une quantité efficace d'un colorant polyquinoïdique choisi dans la catégorie constituée par:

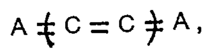

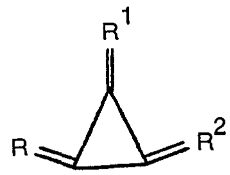

et

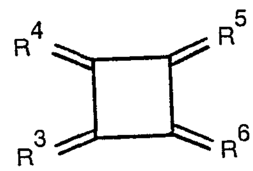

où

A est choisie parmi les groupes quinoïdes et anthraquinoïdes,

$R^2$ est choisi parmi les groupes quinoïdes, les groupes anthraquinoïdes et un atome d'oxygène,

$R^3$ est un groupe quinoïgorie, et

$R^4$, $R^5$ et $R^6$ sont choisis dans la catégorie constituée par les groupes quinoïdes et les atomes d'oxygène, sous réserve qu'au moins un de $R^4$, $R^5$ et $R^6$ soit un groupe quinoîde.

8. Une couche isolante photoconductrice électroniquement active comprenant un liant polymère et un composé selon les revendications 1, 2, 3, 4, 5 ou 6 ou un composé sensiblisé selon la revendication 7.

9. Un élément photoconducteur comprenant, en série, une couche conductrice, une couche génératrice de charge et une couche isolante photoconductrice selon la revendication 8.

10. Un élément photoconducteur comprenant un substrat conducteur et une couche selon la revendication 8, où R est choisi dans la catégorie constituée par un groupe alkyle de 2 à 20 atomes de carbone, un groupe phényle, un groupe naphtyle ou un groupe benzyle.

11. Un procéde de formation d'images comprenant la réalisation d'une charge électrique sur un élément comprenant, en série, une couche conductrice, une couche génératrice de charge photoconductrice et une couche isolante photoconductrice selon la revendication 8, l'irradiation dudit élément chargé avec un motif de rayonnement pour que les zones de ladîte charge soient conduites sélectivement à ladite couche conductrice, et une attraction sélective de matière aux zones chargées ou non chargées dudit élement pour former une image.

12. Le procédé de la revendication, 1 dans lequel ladite image est transférée à une autre surface par contact avec celle-ci.

**Patentansprüche**

1. Elektrisch aktive Donorverbindung der Formel

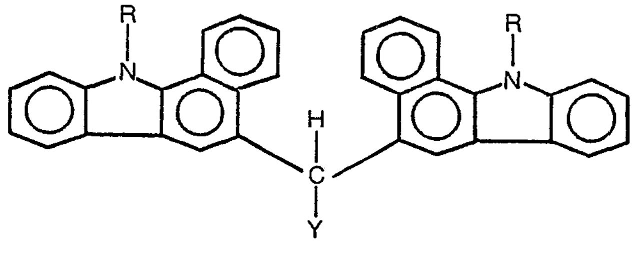

in der R und Y unabhängig voneinander aus der Klasse der aliphatischen, hererozyklischen und gemischten aliphatischaromatischen Gruppen ausgewählt sind, wobei die Bezocarbazolringe nichtsubstituiert oder substituiert sein können.

2. Verbindung nach Anspruch 1, dadurch gekennzeichnet, daß die Benzocarbazolringe nichtsubstituiert sind und R und Y unabhängig voneinander aus der Klasse der aliphatischen, aromatischen und gemischten aliphatisch-aromatischen Gruppen ausgewählt sind.

3. Verbindung nach Anspruch 1, dadurch gekennzeichnet, daß Y eine aromatische Gruppe ist.

4. Verbindung nach Anspruch 1, dadurch gekennzeichnet, daß R eine Alkylgruppe mit 1 bis 20 Kohlenstoffatomen ist.

5. Verbindung nach Anspruch 1, dadurch gekennzeichnet, daß Y eine aromatische Gruppe und R eine Alkylgruppe mit 2 bis 20 Kohlenstoffatomen, eine Phenylgruppe, eine Naphthylgruppe oder eine Benzylgruppe ist.

6. Verbindung nach Anspruch 1, dadurch gekennzeichnet, daß Y eine Phenylgruppe und R eine n-Alkylgruppe mit 2 bis 20 Kohlenstoffatomen ist.

7. Verbindung nach einem der Ansprüche 1 bis 6, dadurch gekennzeichnet, daß sie zu ihrer Sensibilisierung eine wirksame Menge eines Polychinoidfarbstoffes enthält, der ausgewählt ist aus der Klasse der Verbindungen mit den Formeln

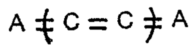,

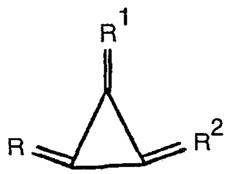

and

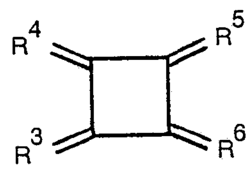

in denen

A aus den Chinoid- und Anthrachinoidgruppen ausgewählt ist,

R und $R^1$ aud den Chinoid- und Anthrachinoidgruppen ausgewählt sind,

$R^2$ aus den Chinoid- und Anthrachinoidgruppen ausgewählt oder ein Sauerstoffatom ist,

$R^3$ eine Chinoidgruppe ist und

$R^4$, $R^5$ und $R^6$ aus der aus den Chinoidgruppen und dem Sauerstoffatom bestehenden Klasse ausgewählt sind, wobei mindestens einer der Bestandteile $R^4$, $R^5$ und $R^6$ eine Sauerstoffgruppe ist.

8. Elektronisch aktive, photoleitfähige Isolierschicht mit einem polymeren Bindemittel und einer Verbindung nach Anspruch 1, 2, 3, 4, 5 oder 6 oder einer sensibilisierten Verbindung nach Anspruch 7.

9. Photoleitendes Element, in dem eine leitende Schicht, eine ladungserzeugende Schicht und eine photoleitende Isolierschicht nach Anspruch 8 nacheinander angeordnet sind.

10. Photoleitendes Element mit einem leitenden Substrat und einer Schicht nach Anspruch 8, dadurch gekennzeichnet, daß R aus der Klasse der Alkylgruppen mit 2 bis 20 Kohlenstoffatomen, der Phenylgruppen, der Naphthylgruppen und der Benzylgruppen ausgewählt ist.

11. Bilderzeugungsverfahren, in dem eine elektrische Ladung auf ein Element aufgebracht wird, in dem eine leitende Schicht, eine ladungserzeugende photoleitende Schicht und eine photoleitende Isolierschicht nach Anspruch 8 nacheinander angeordnet sind, das geladene Element mit einem Strahlungsmuster bestrahlt wird, so daß die Ladung in ausgewählten Flächenbereichen zu der leitenden Schicht geleitet wird, und zum Erzeugen des Bildes eine Ansiehung von material zu ausgewählten geladenen oder ungeladenen Flächenbereichen des Elements veranlaßt wird.

12. Verfahren nach Anspruch 11, dadurch gekennzeichnet, daß das Bild durch Berührung mit einer anderen Fläche auf diese übertragen wird.